# EUROPÄISCHE PATENTSCHRIFT

(11) **EP 1 529 026 B1**
(45) Veröffentlichungstag und Bekanntmachung des Hinweises auf die Patenterteilung: **19.10.2011**
(21) Anmeldenummer: 03792223.4
(22) Anmeldetag: 23.07.2003
(51) Int. Cl.: C07C 67/58, C07C 67/60, C07C 69/80, C07C 69/44

(54) **VERFAHREN ZUR ABTRENNUNG VON VERESTERUNGSKATALYSATOR**
METHOD FOR SEPARATING AN ESTERIFICATION CATALYST
PROCEDE DE SEPARATION D'UN CATALYSEUR D'ESTERIFICATION

(30) Priorität: 08.08.2002 DE 10236279
(43) Veröffentlichungstag der Anmeldung: 11.05.2005
(73) Patentinhaber: BASF SE, 67056 Ludwigshafen (DE)
(72) Erfinder: SCHWIRTEN, Kurt, 67227 Frankenthal (DE); DISTELDORF, Walter, 67157 Wachenheim (DE); GOLFIER, Günther, 67227 Frankenthal (DE); PETERS, Jarren, 68163 Mannheim (DE)
(86) Internationale Anmeldenummer: PCT/EP2003/008035
(87) Internationale Veröffentlichungsnummer: WO 2004/018406

(56) Entgegenhaltungen:
- DE-A- 2 318 657
- DE-A- 2 612 355
- GB-A- 1 030 214
- DATABASE CA [Online] CHEMICAL ABSTRACTS SERVICE, COLUMBUS, OHIO, US; SEDIVY, JOSEF: "Breaking of oil emulsions in water using multivalent cation salts" retrieved from STN Database accession no. 118:45081 XP002262193 & CS 274 812 B (VYZKUMNY USTAV VODOHOSPODARSKY, CZECH.) 12. November 1991 (1991-11-12) in der Anmeldung erwähnt

## Beschreibung

Die vorliegende Erfindung betrifft ein Verfahren zur Abtrennung des Veresterungskatalysators aus einem rohen Weichmacherester, der durch Umsetzung einer Dicarbonsäuren mit C₈- bis C₁₃-Alkoholen erhalten wurde, durch Behandlung des Rohesters mit einer wäßrigen Alkali-Lösung bei Temperaturen im Bereich von 10 bis 100°C und anschließender Abtrennung der wäßrig-alkalischen, den hydrolysierten Veresterungskatalysator enthaltenden Phase, durch Schwerkraft-Phasenscheidung.

Phthalsäurediester und Adipinsäurediester sind wichtige Weichmacher für Kunststoffe, insbesondere PVC. Bei der Herstellung dieser Diester im industriellen Maßstab wird in der Regel Phthalsäureanhydrid bzw. Adipinsäure mit der ca. doppelten molaren Menge eines Alkohols in Gegenwart eines Veresterungskatalysators umgesetzt. Wichtige Vertreter der so erhältliche Klasse der Phthalsäurediester und Adipinsäurediester sind jene Dialkylester, welche sich von den entsprechenden Alkanolen, beispielsweise Octanolen oder Nonanolen, ableiten. Als Veresterungskatalysatoren kommen dabei heutzutage meist lewis-saure Salze von Elementen der IV. Hauptgruppe oder der IV. Nebengruppe des periodischen Systems der Elemente zum Einsatz.

Bei Ende der Veresterungsreaktion wird der Veresterungskatalysator normalerweise durch Zugabe von Alkali zerstört. Hierzu verwenet man aus praktischen Erwägungen zumeist wäßrige Lösungen eines Alkalimetallhydroxids oder -carbonats. Die Hydrolyseprodukte des Veresterungskatalysators finden sich am Ende der Katalysatorzerstörung überwiegend in der wäßrigen Phase, welche dann abgetrennt wird.

Jedoch ist die Trennung von organischer Produktphase und alkalischer Wasserphase in der Praxis meist **dadurch erschwert, dass** sich am Ende der Katalysatorzerstörung eine Emulsionsschicht zwischen den beiden zu trennenden Phasen ausbildet.

Es wurde nunmehr festgestellt, dass diese Emulsionsschicht Hydrolyseprodukte des Katalysators sowie Alkalisalze von Halbestern aus einem Mol Dicarbonsäure und einem Mol Veresterungsalkohol (im folgenden "Halbester" genannt) enthält. Die Emulsionsschicht enthält häufig auch noch beträchtliche Mengen Diester.

Die Halbester gehen zum einen zurück auf eine unvollständige Umsetzung des Monoesters mit dem Veresterungsalkohol. Zum anderen können sie auch aus Diester durch alkalische Hydrolyse bei der Zerstörung des Veresterungskatalysator mittels Alkali entstehen.

Mit längeren Verweilzeiten in üblichen Trennbehältern gelingt es in der Regel nicht, derartige Emulsionen zu brechen. Das hat zur Folge, dass die Emulsionen häufig bei der Phasentrennung teilweise in die organischen Phase gelangen und bei deren Aufarbeitung zu störenden Ablagerungen in den nachgeschalteten Anlagenteilen der Weichmacherproduktion führen oder teilweise mit der wäßrigen Phase abgeschieden werden, was zu Produktverlusten führt.

Gesucht war demnach ein Verfahren, derartige Emulsionen vor der Phasentrennung zu "brechen".

Dem Fachmann ist zur Erreichung dieses Zweckes bereits die Verwendung chemischer Mittel bekannt.

DE-A 2612355 beschreibt ein Verfahren zur Abtrennung von Katalysatoren aus rohen Weichmacherestern, die durch Veresterung von Phthalsäureanhydrid oder Adipinsäure mit C₆ bis C₁₃-Alkoholen unter Verwendung von Veresterungskatalysatoren der Zinn-, Titan- oder Zirkon-Reihe erhalten wurden, durch Behandlung der rohen Ester mit wässrigem Alkali bei Temperaturen unter 100°C und anschließendes Abtrennen der wässrigen, den hydrolysierten Katalysator enthaltenden Phase durch Dekantieren, wobei man den neutralen oder schwach sauren Rohester mindestens 3 Minuten mit 5 bis 20 Gewichtsteilen, bezogen auf 100 Gewichtsteile des rohen Esters, einer 0,1 bis 5 gewichtsprozentigen wässrigen Alkalilösung so vermischt, dass eine Emulsion entsteht, in der die Tropfen der organischen Phase einen Durchmesser von 0,5 bis 10 mm aufweisen.

GB-A 1030214 betrifft ein Verfahren zur Reinigung polymerer Weichmacher. Diese polymeren Weichmacher neigen beim Vermischen mit wässriger Alkalilauge zur Bildung stabiler Emulsionen, die sich nur langsam oder gar nicht in eine wässrige und eine organische Phase trennen lassen. Zwecks Vermeidung der Bildung solcher Emulsionen im Zuge der Neutralisation des aciden Veresterungskatalysators wird zur Neutralisation eine möglichst geringe Menge konzentrierter Alkalilauge eingesetzt. Die dabei ausfallenden unlöslichen Alkalisalze werden durch Filtration vom Weichmacherester abgetrennt.

So beschreibt die DE-A 23 18 657 die zweistufige Zerstörung einer Öl-in-Wasser-Emulsion durch Einstellen eines pH-Wertes auf 2 bis 3 anhand von FeCl₃ oder AlCl₃. Unmittelbar im Anschluss daran wird mit NaOH oder Ca(OH)₂ neutralisiert.

Das tschechische Patent CS 274812 schlägt ebenfalls ein zweistufiges Verfahren vor, bei dem die Emulsion zunächst angesäuert und daraufhin mit einem mehrwertigen Salz, beispielsweise Al₂(SO₄)₃, Fe₂(SO₄)₃ oder FeCl₃ behandelt wird.

Diese bekannten Verfahren setzten demnach jeweils voraus, dass die zu brechende Emulsion einen pH-Wert von deutlich unter 7 (saurer Bereich) aufweist.

Für die Anwendung auf das vorliegende technische Problem, nämlich eine Emulsion an der Grenzfläche zwischen einer organischen Produktphase und einer alkalischer Wasserphase eines Veresterungsrohproduktes zu zerstören, sind diese bekannten Verfahren umständlich und wenig wirtschaftlich.

Der vorliegenden Erfindung lag daher die Aufgabe zugrunde, ein in der Durchführung vereinfachtes und wirtschaftlicheres Verfahren zur Zerstörung von solchen Emulsionen bereit zu stellen, wie sie bei der alkalisch-wäßrigen Hydrolyse der Katalysatoren im oben genannten Herstellverfahren für Dicarbonsäurediester.

Demgemäß wurde ein Verfahren zur Abtrennung des Veresterungskatalysators aus einen rohen Weichmacherester, der durch Umsetzung einer Dicarbonsäuren mit C₈- bis C₁₃-Alkoholen erhalten wurde, gefunden, durch Behandlung des Rohesters mit einer wäßrigen Alkali-Lösung bei Temperaturen im Bereich von 10 bis 100°C und anschließender Abtrennung der wäßrig-alkalischen, den hydrolysierten Veresterungskatalysator enthaltenden Phase, durch Schwerkraft-Phasenscheidung, welches dadurch gekennzeichnet ist, dass man den Rohester vor oder während der Phasentrennung mit einem Salz eines zwei- oder mehrwertigen Metalls oder Gemischen aus solchen Salzen behandelt.

Als Dicarbonsäuren kommen aromatische und aliphatische organische Dicarbonsäuren mit vorzugsweise 3 bis 12 Kohlenstoffatomen in Betracht. Besonders bevorzugt sind derartige Dicarbonsäuren mit 6 bis 8 Kohlenstoffatomen wie Adipinsäure und Phthalsäure. An Stelle der Dicarbonsäuren können auch deren bei Veresterungsreaktionen in der Regel reaktiveren Derivate wie Anhydride, im Falle der Phthalsäure etwa das Phthalsäureanhydrid, oder die Säuredichloride zum Einsatz kommen.

Als Veresterungsalkohole kommen vorzugsweise Monoalkohole mit 6 bis 13 Kohlenstoffatomen zum Einsatz. Besonders bevorzugt sind verzweigte, unverzweigte oder Gemische aus verzweigten und unverzweigten Monoalkanolen mit 8 bis 11 Kohlenstoffatomen.

Das erfindungsgemäße Verfahren ist besonders geeignet für die Zerstörung von Emulsionen, wie sie bei der Herstellung von Phthalsäure-di-C₈-estern, Phthalsäure-di-C₉-estern, Phthalsäure-di-C₁₀-estern, Adipinsäure-di-C₈-estern, Adipinsäure-di-C₉-estern und Adipinsäure-di-C₁₀-estern auftreten.

Als Veresterungskatalysator ist eine lewis-saure Verbindung eines Elements der IV. Hauptgruppe oder der IV. Nebengruppe des periodischen Systems der Elemente bevorzugt.

Bevorzugte Veresterungskatalysator-Metalle sind Zinn, Antimon, Titan oder Zirkonium; besonders bevorzugt ist Titan.

Bevorzugte Veresterungskatalysatoren sind die Halogenide, Alkoxylate und Alkoxycarbonylate der Veresterungskatalysator-Metalle, beispielsweise TiCl₄, ZrCl₂(OEthyl)₂, ZrOCl₂; besonders bevorzugt sind die Titanalkoxylate und insbesondere Ti(OEthyl)₄, Ti(O-iso-Propyl)₄, Ti(O-iso-Butyl)₄.

Bezogen auf die zu veresternde Dicarbonsäure oder gegebenenfalls das Derivat der Dicarbonsäure werden in der Regel 0,001 bis 0,8, vorzugsweise 0,01 bis 0,08 Gew.-% des Veresterungskatalysators eingesetzt.

Die Herstellung der Dicarbonsäurediester unter Verwendung solcher Veresterungskatalysatoren ist dem Fachmann im Übrigen hinreichend geläufig (vgl. z.B. DE-A 1 945 359), so dass sich nähere Ausführungen hierzu erübrigen.

Nach erfolgter Umsetzung der Dicarbonsäure mit dem Veresterungsalkohol in Gegenwart des Veresterungskatalysators liegt in der Regel eine homogene Lösung des Veresterungskatalysators im überwiegend aus Diester bestehenden rohen Produktgemisch vor.

Zur Zerstörung des Veresterungskatalysators wird das Produktgemisch in der Regel mit einer 2- bis 10 Gew.-%-igen wäßrigen Alkalilösung wie Natronlauge oder einer wäßrigen Lösung von Kaliumcarbonat und Wasser behandelt. Dabei wird normalerweise so gearbeitet, dass am Ende das Phasenverhältnis der wäßrigen Phase zu der organischen Phase im Bereich von 0,1 : 1 bis 10 : 1 Gewichtsteile liegt.

Dem so erhaltenen Zweiphasensystem aus organischer Produktphase und wäßriger Phase werden, bezogen auf das Volumen der wäßrigen Phase, pro Liter 0,05 bis 30 mmol eines Salzes eines zwei- oder mehrwertigen Metalls oder eines Gemischs aus solchen Salzen zugesetzt.

Für das erfindungsgemäße Verfahren werden als Salze vorzugsweise verwendet: Halogenide, Sulfate, gemischte Halogenidsulfate und ternäre Alkalihalogenide und -sulfate von Eisen, Aluminium und Calcium beispielsweise Sulfate wie Aluminiumsulfate, z.B. Al₂(SO₄)₃, Eisensulfate, z.B. Fe₂(SO₄)₃, Chloride wie Aluminiumchlorid, Eisen(III)chlorid, Kalium-Aluminiumsulfate wie KAl(SO₄)₂, Calciumsalze wie Calciumoxid und Calciumchlorid. Besonders bevorzugt sind Calcium- und Aluminiumsalze, und hierunter sind die Aluminiumsalze ganz besonders bevorzugt.

Die Zugabe des Salzes oder des Gemisches von Salzen kann in Form einer Lösung, vorzugsweise in Wasser, oder als Feststoff erfolgen.

In einer bevorzugten Ausführungsform des erfindungsgemäßen Verfahrens wird das Salz oder das Gemisch aus den Salzen dem Rohester während der Alkalibehandlung und/oder einer gegebenenfalls durchgeführten Wäsche zugesetzt.

Die Umsetzung der Metallsalzlösung wird im Allgemeinen bei einer Temperatur von 10 bis 120, vorzugsweise 40 bis 90°C und bei einem Druck von 0,5 bis 4 bar, vorzugsweise bei Normaldruck durchgeführt.

Das erfindungsgemäße Verfahren kann kontinuierlich oder diskontinuierlich durchgeführt werden, wobei es in der Regel besonders wirtschaftlich ist, dort kontinuierlich zu arbeiten, wo auch die Zerstörung des Veresterungskatalysators kontinuierlich durchgeführt wird. Entsprechende Überlegungen gelten für eine diskontinuierliche Arbeitsweise.

Vorzugsweise kommen als Mischvorrichtungen Rührbehälter in Betracht, die mit stufenlos regelbaren Getrieben ausgerüstet sind, beispielsweise Blattrührer, die eine homogene Durchmischung bewirken.

Die mittlere Verweilzeit in der Mischvorrichtung beträgt 0,5 bis 45 Minuten und bevorzugt 10 bis 20 Minuten.

Der Rohester hat vorzugsweise vor der Schwerkraft-Phasenscheidung einen Gehalt an Dicarbonsäurehalbester-monosalz von 0,1 bis 5 Gew.-%.

Bei der anschließenden Phasentrennung unter dem Einfluß der Schwerkraft können stehende Phasenscheider verwendet werden, da hier die Abtrennung der Feststoffe (z.B. Titanhydroxid) besonders einfach ist. Es können aber auch liegende Phasenscheider verwendet werden, da die Titanhydroxide rasch und vollständig in der wäßrigen Phase sedimentieren und sich dabei normalerweise keine schwer auftrennbare feststoffhaltige Zwischenschicht aus Rohester, wäßriger Phase und Titanhydroxid-Partikel bildet.

Die Phasentrennzeiten liegen für die Flüssig/Flüssig-Trennung und für die Fest/Flüssig-Trennung in der Regel im Bereich von 5 bis 30 Minuten.

Die abgetrennte organische Phase, die im Wesentlichen aus dem Diester und unumgesetztem Veresterungsalkohol besteht, wird im Weiteren in dem Fachmann an sich bekannter Weise weiter gereinigt, etwa durch Ausdämpfen.

Die abgetrennte wäßrige Phase enthält normalerweise die Hauptmenge des Dicarbonsäurehalbester-monosalzes.

Ein Vorteil des erfindungsgemäßen Verfahrens besteht darin, dass die Phasenscheidung in der Regel rasch und störungsfrei (ohne Ausbildung von feststoffhaltigen Zwischenschichten und Emulsionen) erfolgt.

Als besonderer Vorteil ist zu werten, dass die Weichmacherester feststofffrei in die nachfolgenden Verfahrensstufen gelangen. Hierdurch können die Standzeiten der verwendeten Apparate auf das bis zu 10- bis 20-fache erhöht werden.

Die wirtschaftlichen Vorteile bei der Ausübung des erfindungsgemäßen Verfahrens im technischen Maßstab bestehen in einer Kapazitätserhöhung in den Verfahrensstufen der Rohesterbehandlung sowie in der Erhöhung der Verfügbarkeit und der Betriebssicherheit. Durch die Abnahme der Zahl der Reinigungsphasen werden die Wartungskosten der verwendeten Anlagen erheblich reduziert und die Weichmacherester-Verluste verringert.

### Beispiele

### Beispiel 1

180 g Diisononylphthalat-Rohester (hergestellt gemäß DE-A 197 21 347) wurden in einem Scheidetrichter mit 20 g Wasser und 0,4 Gew.-%, bezogen auf den Ansatz, des Mono-Natriumsalzes von Monoisononylphthalat 5 Minuten lang gründlich vermischt. Danach war die Mischung milchig trübe, und eine Phasengrenze war nicht mehr erkennbar. Zu der Mischung wurden anschließend 3 Gew.-%, bezogen auf den Ansatz, einer 0,5 millimolaren wäßrigen Lösung von Aluminiumsulfat zugegeben und die gesamte Mischung erneut kräftig durchmischt. Nach zehnminütigem Stehenlassen war eine Phasengrenze zwischen den zwei unterschiedlichen trüben Phasen klar zu erkennen.

### Beispiel 2

Eine stabile Emulsion aus einer Produktionsanlage mit 24 Gew.-% Wasser vom pH 9,8, 2,5 Gew.-% Isononanol und 73 Gew.-% Diisononylphthalat sowie 0,5 Gew.-% Mono-Natriumsalz von Monoisononylphthalat wurde mit 8 Gew.-%, bezogen auf den Ansatz, einer 0,5 millimolaren wäßrigen Lösung von Aluminiumsulfat versetzt und kräftig durchmischt. Nach zehnminütigem Stehenlassen war eine Phasengrenze zwischen den zwei unterschiedlichen Phasen klar zu erkennen.

### Beispiel 3

Drei Schütteltrichter (Schütteltrichter 1, 2 und 3) wurden mit je 25 ml Wasser und je 250 g Diisononylphthalat befüllt und in jeden Scheidetrichter 0,2 g des Mono-Natriumsalzes von Monononylphthalat gegeben. Alle drei Schütteltrichter wurden danach kräftig durchgeschüttelt. Die Phasengrenze war danach verschwunden und es lag in allen drei Fällen eine stabile Emulsion vor. Es ging wie folgt weiter:

Schütteltrichter 1: Nach 1 h 30 min lag immer noch eine weiße Emulsion vor, nur hatten sich unten ca. 5 ml wäßrige Phase abgeschieden.

Schütteltrichter 2: Es wurden 5 ml einer 10 mmolaren Calciumchlorid-Lösung zugesetzt, und der Inhalt des Schütteltrichters wurde erneut durchmischt. Nach 15 min bildeten sich im unteren Bereich größere Tropfen. Nach 1 h hatten sich 30 ml wäßrige Phase abgeschieden. Oberhalb der Phasengrenze lag eine 8-10 mm dicke weiße undurchsichtige Schicht vor, während die weitere organische Phase zwar eingetrübt, aber durchsichtig war. Nach 1 h 30 min war keine weitere Veränderung feststellbar.

Schütteltrichter 3: Es wurden 30 ml einer 10 molaren Calciumchlorid-Lösung zugesetzt und der Inhalt des Schütteltrichters wurde erneut durchmischt. Nach 15 min war eine deutliche Phasengrenze feststellbar. Nach 30 min hatten sich 55 ml wäßrige Phase abgeschieden. Es lag eine scharfe Phasengrenze - ohne Andeutung einer Emulsion - vor.

## Patentansprüche

1. Verfahren zur Abtrennung des Veresterungskatalysators aus einem rohen Weichmacherester, der durch Umsetzung einer Dicarbonsäuren mit C₈- bis C₁₃-Alkoholen erhalten wurde, durch Behandlung des Rohesters mit einer wäßrigen Alkali-Lösung bei Temperaturen im Bereich von 10 bis 100°C und anschließender Abtrennung der wäßrig-alkalischen, den hydrolysierten Veresterungskatalysator enthaltenden Phase, durch Schwerkraft-Phasenscheidung, **dadurch gekennzeichnet, dass** man den Rohester vor oder während der Phasentrennung mit einem Salz eines zwei- oder mehrwertigen Metalls oder Gemischen aus solchen Salzen behandelt.

2. Verfahren nach Anspruch 1, **dadurch gekennzeichnet, dass** man als Veresterungskatalysator eine lewis-saure Verbindung eines Elements der IV. Hauptgruppe oder der IV. Nebengruppe des periodischen Systems der Elemente verwendet.

3. Verfahren nach Anspruch 1 oder 2, **dadurch gekennzeichnet, dass** man als Veresterungskatalysator eine Verbindung des Titans verwendet.

4. Verfahren nach den Ansprüchen 1 bis 3, **dadurch gekennzeichnet, dass** der Rohester vor der Schwerkraft-Phasenscheidung einen Gehalt an Dicarbonsäurehalbester-monosalz von 0,1 bis 5 Gew.-% aufweist.

5. Verfahren nach den Ansprüchen 1 bis 4, **dadurch gekennzeichnet, dass** man als Salz eines zwei- oder mehrwertigen Metalls ein Calcium- oder Aluminiumsalz verwendet.

6. Verfahren nach Anspruch 5, **dadurch gekennzeichnet, dass** man ein Aluminiumsalz verwendet.

7. Verfahren nach Anspruch 6, **dadurch gekennzeichnet, dass** man das Aluminiumsalz in einer Menge von 0,05 bis 30 mmol pro Liter der wäßrig-alkalischen Phase einsetzt.

## Claims

1. A process for removal of the esterification catalyst by separation from a crude plasticizer ester obtained by reacting a dicarboxylic acid with C₈-C₁₃ alcohols, by treating the crude ester with an aqueous alkali solution in the range from 10 to 100°C and then separating the aqueous alkaline phase comprising the hydrolyzed esterification catalyst by gravitational phase separation, which comprises treating the crude ester, prior to or during the phase separation, with a salt of a di- or polyvalent metal, or with a mixture of these salts.

2. The process according to claim 1, wherein the esterification catalyst used comprises a Lewis-acid compound of an element of the 4th main group or of the 4th transition group of the Periodic Table of the Elements.

3. The process according to claim 1 or 2, wherein the esterification catalyst used comprises a compound of titanium.

4. The process according to any of claims 1 to 3, wherein, prior to the gravitational phase separation, the crude ester has a content of from 0.1 to 5% by weight of monosalt of dicarboxylic half-ester.

5. The process according to any of claims 1 to 4, wherein the salt used of a di- or polyvalent metal comprises a calcium salt or aluminum salt.

6. The process according to claim 5, wherein use is made of an aluminum salt.

7. The process according to claim 6, wherein the amount of aluminum salt used is from 0.05 to 30 mmol per liter of the aqueous alkaline phase.

## Revendications

1. Procédé pour la séparation du catalyseur d'estérification d'un ester plastifiant brut, qui a été obtenu par transformation d'un acide dicarboxylique avec des alcools en C₈-C₁₃, par traitement de l'ester brut avec une solution aqueuse d'alcali à des températures dans la plage de 10 à 100°C et séparation consécutive de la phase aqueuse-alcaline, contenant le catalyseur d'estérification hydrolysé, par séparation des phases par gravité, **caractérisé en ce qu'**on traite l'ester brut avant ou pendant la séparation des phases avec un sel d'un métal divalent ou polyvalent ou des mélanges de ces sels.

2. Procédé selon la revendication 1, **caractérisé en ce qu'**on utilise comme catalyseur d'estérification un composé acide de Lewis d'un élément du IVème groupe principal ou du IVème groupe secondaire du système périodique des éléments.

3. Procédé selon la revendication 1 ou 2, **caractérisé en ce qu'**on utilise un composé du titane comme catalyseur d'estérification.

4. Procédé selon les revendications 1 à 3, **caractérisé en ce que** l'ester brut présente, avant la séparation des phases par gravité, une teneur en monosel du semi-ester de l'acide dicarboxylique de 0,1 à 5% en poids.

5. Procédé selon les revendications 1 à 4, **caractérisé en ce qu'**on utilise comme sel d'un métal divalent ou polyvalent un sel de calcium ou d'aluminium.

6. Procédé selon la revendication 5, **caractérisé en ce qu'**on utilise un sel d'aluminium.

7. Procédé selon la revendication 6, **caractérisé en ce qu'**on utilise le sel d'aluminium en une quantité de 0,05 à 30 mmoles par litre de la phase aqueuse-alcaline.
